# FASCICULE DE BREVET EUROPEEN

(11) **EP 1 602 357 B1**
(45) Date de publication et mention de la délivrance du brevet: **08.10.2008**
(21) Numéro de dépôt: 05290845.6
(22) Date de dépôt: 15.04.2005
(51) Int. Cl.: A61K 8/34, A61K 8/58, A61Q 13/00

(54) **Composition de parfum à base de décaméthyltétrasiloxane**
Parfümzusammensetzung enthalend decamethyltetrasiloxane
Perfum composition comprising decamethyltetrasiloxane

(30) Priorité: 29.04.2004 FR 0450824
(43) Date de publication de la demande: 07.12.2005
(73) Titulaire: L'ORÉAL, 75008 Paris (FR)
(72) Inventeur: Bara, Isabelle, 94210 La Varenne st Hilaire (FR); Buet, Daniel, 94240 L'Hay les Roses (FR)
(74) Mandataire: Renard, Emmanuelle

(56) Documents cités:
- EP-A1- 0 566 240
- US-A- 5 665 339
- US-B1- 6 403 109

## Description

La présente invention se rapporte à une nouvelle composition de parfum comprenant : (a) de 11 à 60% en poids d'un mélange de matières odoriférantes, (b) de 51 à 80% en poids d'éthanol, (c) de 3 à 30% en poids de décaméthyltétrasiloxane, par rapport au poids total de la composition.

Les compositions de parfum comprennent habituellement un mélange de matières odoriférantes ayant une tension de vapeur inférieure à la pression atmosphérique à 25°C et qui sont généralement liquides à 25°C, mais parfois également solides, dans un milieu physiologiquement acceptable à base d'éthanol et éventuellement d'eau.

L'éthanol constitue un bon solubilisant des ingrédients parfumants, et présente en outre l'avantage d'être peu coûteux et de permettre la formulation de compositions transparentes. En revanche, il a l'inconvénient de réagir chimiquement avec les oxydes d'azote de l'atmosphère pour former de l'ozone, et constitue à ce titre une source de pollution atmosphérique que l'on cherche à éviter.

Il est donc habituel de remplacer une partie de l'éthanol par de l'eau. Toutefois, l'eau et l'éthanol ne sont pas chimiquement inertes et réagissent avec les composés des parfums en créant au cours du temps des altérations olfactives et/ou des modifications de l'aspect des liquides (jaunissement, brunissement). Les réactions chimiques responsables de ces dégradations sont principalement :
- la formation d'acétals, par condensation de l'éthanol avec les composés chimiques à fonction aldéhyde,
- la saponification ou hydrolyse des composés à fonction ester,
- l'oxydation des mono-terpènes ou sesquiterpènes présents particulièrement dans les extraits végétaux, et
- l'oxydation de l'éthanol en acétaldéhyde puis en diéthylacétal (DEA) de l'acétaldéhyde, accompagnée d'une formation d'acide acétique puis d'acétate d'éthyle.

En outre, l'eau n'est pas un bon solvant des parfums et diminue le pouvoir solvant de l'éthanol. Des problèmes de troubles ou de dissolution apparaissent parfois dès le début de la formulation des parfums et obligent à réduire significativement le taux de parfum. Il en résulte des parfums plus dilués, alors que la tendance actuelle est de réaliser des parfums plutôt puissants et fortement concentrés (plus de 10% de matières parfumantes).

On comprend donc l'intérêt qu'il y a à substituer l'eau présente dans les parfums par une autre matière première inerte, volatile, ayant une bonne miscibilité avec l'éthanol et permettant de solubiliser des quantités importantes d'ingrédients parfumants.

Or, la Demanderesse a découvert qu'un polydiméthylsiloxane particulier, le décaméthyltétrasiloxane, permettait de satisfaire ce besoin en présence d'une forte concentration d'éthanol. Les compositions de parfum ainsi obtenues permettent de réaliser un compromis économiquement acceptable en conservant une quantité importante d'éthanol, qui est bon marché, sans perte de stabilité ni altération des caractéristiques olfactives du parfum au cours du temps.

On connaît du brevet US-5,665,339 des compositions de parfums comprenant au plus 10% de parfum, de 10 à 90% d'éthanol, de 10 à 90% d'un polydiméthylsiloxane tel que le décaméthyltétrasiloxane, et moins de 1% d'eau. Toutefois, le polydiméthylsiloxane est utilisé dans ce document pour améliorer la cosméticité et diminuer l'irritation provoquée par les lotions alcooliques. Il n'est pas suggéré qu'il permette la formulation de compositions de parfums renfermant plus de 10% d'ingrédients parfumants.

On connaît par ailleurs de la demande WO 99/01106 des compositions destinées à parfumer des textiles sans les tacher, renfermant de 0,1 à 90% de parfum ; de 10 à 99,9% d'une silicone volatile telle que le décaméthyltétrasiloxane ; éventuellement de l'éthanol (au plus 10%) et moins de 1% d'eau. Ces compositions ne renferment pas suffisamment d'éthanol pour permettre une bonne solubilisation du parfum à un coût économiquement raisonnable et peuvent en outre être responsables d'une pénétration indésirable du parfum dans la peau.

Le brevet US-5,160,494 divulgue des compositions de parfum anhydres renfermant de 5 à 30% de parfum ; de 1 à 95% d'alkylméthyldisiloxane, destiné à remplacer l'éthanol qui peut tout de même représenter de 0 à 90% du poids de la composition ; et éventuellement de 0 à 40% de silicones telles que le décaméthyltétrasiloxane. Ce document ne suggère pas que le décaméthyltétrasiloxane puisse permettre d'abaisser le taux d'alcool tout en le maintenant à un taux économiquement acceptable et sans nécessiter obligatoirement le recours à un alkylméthyldisiloxane coûteux.

Les demandes US 2003/0190267 et US 2003/0190255 divulguent des compositions de parfum d'ambiance ne générant pas de dépôts, qui comprennent de 0,01 à 50%, de préférence de 15 à 25% d'éthanol ; de 20 à 30% (respectivement, de 14 à 40%) d'une silicone telle que le décaméthyltétrasiloxane ; et au plus 5% d'eau. Les exemples donnés dans ces demandes contiennent plus de 10% de parfums. Il n'est pas suggéré dans ces demandes de formuler des compositions de parfum renfermant plus de 50% d'éthanol, qui risqueraient au contraire de poser des problèmes de sécurité eu égard à leur inflammabilité, s'agissant de parfums d'ambiance. En outre, il n'est pas décrit de composition ayant un point éclair voisin de 20°C, dans la mesure où il est plutôt voisin de 60°C.

La présente invention a donc pour objet une composition de parfum comprenant : (a) de 11 à 60% en poids d'un mélange de matières odoriférantes, (b) de 51 à 80% en poids d'éthanol, (c) de 3 à 30% en poids de décaméthyltétrasiloxane, par rapport au poids total de la composition.

Les matières odoriférantes contenues dans la composition de parfum selon l'invention sont des composés usuellement utilisés par les parfumeurs et ils sont notamment décrits dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III. USA.

Il peut s'agir de produits naturels (huiles essentielles, absolus, résinoïdes, résines, concrètes) et/ou synthétiques (hydrocarbures, alcools, aldéhydes, cétones, éthers, acides, esters, acétals, cétals, nitriles, saturés ou insaturés, aliphatiques ou cycliques).

Des exemples d'huiles essentielles comprennent les huiles essentielles de citron, d'orange, d'anis, de bergamote, de rose, de géranium, de gingembre, de néroli, de basilic, de romarin, de cardamome, de camphre, de cèdre, de camomille, de santal, de sauge, et leurs mélanges, sans que cette liste soit limitative.

Des exemples d'autres composés odoriférants sont notamment : le géraniol, l'acétate de géranyle, le farnésol, le bornéol, l'acétate de bornyle, le linalol, l'acétate de linalyle, le propionate de linalyle, le butyrate de linalyle, le tétrahydrolinalol, le citronellol, l'acétate de citronellyle, le formate de citronellyle, le propionate de citronellyle, le dihydromyrcenol, l'acétate de dihydromyrcenyle, le tétrahydromyrcenol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, l'acétate d'isononyle, l'acétate de vétivéryle, le vétivérol, l'alpha -hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyltétrahydropyrane, la 2-n-heptylcyclopentanone, la 3-méthyl-2-pentyl-2-cyclopentènone, la menthone, la carvone, la tagétone, la géranyl acétone, le n-décanal, le n-dodécanal, le 9-décènol-1, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, la damascone, les ionones, les méthylionones, les isométhylionones, la solanone, les irones, le cis-3-hexènol et ses esters, les muscs-indane, les muscs-tétraline,les muscs-isochromane, les cétones macrocycliques, les muscs-macrolactones, le brassylate d'éthylène et leurs mélanges.

La composition selon la présente invention renferme de 11 à 60% en poids, et de préférence de 11 à 25% en poids, de matières odoriférantes.

Elle renferme également de l'éthanol et du décaméthyltétrasiloxane, généralement en quantité suffisante pour solubiliser ces matières odoriférantes et conférer à la composition un point éclair supérieur ou égal à 15°C et de préférence inférieur ou égal à 60°C. Ainsi, la quantité d'éthanol représente de 51 à 80%, et de préférence de 51 à 65% du poids de la composition, la quantité de décaméthyltétrasiloxane représentant de 3 à 30%, et de préférence de 10 à 30% du poids de la composition.

Le décaméthyltétrasiloxane est notamment disponible auprès de la société DOW CORNING sous la dénomination commerciale DC 200 Fluid 1.5 cSt.

La composition de parfum selon l'invention comprend aussi en général des solvants, adjuvants ou additifs couramment utilisés dans le domaine de la parfumerie qui ne nuisent pas à l'effet olfactif recherché.

Ainsi, la composition selon l'invention peut contenir une faible quantité d'eau, ne dépassant généralement pas 7% du poids total de la composition, qui peut notamment être apportée par l'éthanol, ce dernier étant en général commercialisé sous la forme d'un mélange d'alcool dénaturé et d'eau.

En outre, selon une forme d'exécution préférée, la composition selon l'invention ne contient pas d'alkyldiméthylsiloxane dont le groupe alkyle contient de 2 à 13 atomes de carbone.

En outre, la stabilité de la composition selon l'invention est telle qu'elle ne requiert pas la présence d'un agent tensioactif. Ainsi, selon une forme d'exécution préférée de l'invention, cette composition renferme moins de 1% en poids, voire moins de 0,5% en poids, voire pas du tout, de tensioactif.

Cette composition de parfum peut constituer une eau de Cologne, une eau de toilette, un parfum ou une lotion après-rasage.

En fonction de son point éclair, elle peut être conditionnée dans un atomiseur ou éventuellement dans un dispositif aérosol. Dans ce dernier cas, la composition selon l'invention contient en outre un gaz propulseur tel que le diméthyléther, le propane, le n-butane, l'isobutane, le pentane, le trichlorofluorométhane, le dichlorodifluorométhane, le chlorodifluorométhane, le 1,1,1,2-tétrafluoroéthane, le chloropentafluoroéthane, le 1-chloro-1,1-difluoroéthane, le 1,1-difluoroéthane, et leurs mélanges. On utilise de préférence le diméthyl éther, l'isobutane et le 1,1,1,2-tétrafluoroéthane, et préférentiellement l'isobutane.
L'invention sera maintenant illustrée par les exemples non limitatifs suivants. Dans ces exemples, les quantités sont indiquées en pourcentage pondéral.

### EXEMPLES

### Exemple 1 : Etude comparative des propriétés de différentes compositions de parfum siliconées

On a testé quatre compositions de parfum pour évaluer leur point éclair et leurs propriétés cosmétiques.

Pour mesurer le point éclair, chaque composition testée est chauffée dans une coupe fermée de dimensions normalisées à une température d'environ 3°C inférieure au point éclair supposé, pendant 60 secondes. Puis une flamme de dimension normalisée est présentée dans les vapeurs de la coupe par une ouverture coulissante. L'essai est répété de 1°C en 1°C. La plus basse température à laquelle se produit l'inflammation est notée comme étant le point éclair. L'essai est réalisé sur un appareil SETAFLASH selon la norme ISO 3679.

Les quatre compositions testées renfermaient chacune : 12% de parfum ; 22% d'un polydiméthylsiloxane (PDMS) dont la nature variait selon les compositions ; et 66% d'éthanol à 93,5% en poids d'alcool dénaturé.

Le tableau ci-dessous rassemble les résultats obtenus pour les quatre compositions testées.

| Composition | PDMS | Point éclair | Propriétés cosmétiques |
|---|---|---|---|
| A | L2 (hexaméthyldisiloxane) | -5°C | satisfaisantes |
| B | L3 (octaméthyltrisiloxane) | 13°C | satisfaisantes |
| C | L4 (décaméthyltétrasiloxane) | 17°C | satisfaisantes |
| D | L5 (dodécaméthylpentasiloxane) | 17°C | effet huileux |

Comme il ressort de ce tableau, seule la silicone de type L4 permet d'obtenir, en association avec l'éthanol une composition ayant un point éclair permettant sa mise en oeuvre industrielle, tout en ayant de bonnes propriétés sensorielles.

### Exemple 2 : Etude comparative de la stabilité de compositions de parfum

On a testé la stabilité au cours du temps de trois compositions de parfum :
- la composition C ci-dessus dans laquelle le parfum correspondait à l'extrait de parfum "Volupté" de Gloria Vanderbilt
- une composition C' contenant 12% du même parfum ; 66% d'éthanol à 93,5% en poids d'alcool dénaturé ; et 22% d'eau à la place du décaméthyltétrasiloxane
- une composition C" correspondant à la composition C' a laquelle a été ajouté 0,05% de BHT comme anti-oxydant.

### Aspects visuel et olfactif :

Après deux mois de conservation à température ambiante et à 45°C, la composition C présentait une forte dégradation olfactive et un brunissement important. La composition C" présentait une dégradation olfactive moyenne et un brunissement toujours important. Au contraire, la composition C conservait même après six mois de conservation à température ambiante et à 45°C un aspect limpide, incolore et homogène et une excellente stabilité olfactive.

### Stabilité chimique :

Les compositions C, C' et C" ont été analysées après trois mois mois à 45°C. On a observé pour la composition C :
- une formation réduite de diéthylacétals, se traduisant par cinq fois moins d'hydroxycitronellal DEA formé, par rapport à la composition C',
- une quasi-absence de linalol, géraniol et nérol, produits de dégradation de l'acétate de linalyle, alors que les compositions C' et C" subissaient environ 50% de dégradation de l'acétate de linalyle,
- une quasi-absence d'alcool benzylique et de benzaldéhyde, produits de dégradation de l'acétate de benzyle, alors que les compositions C' et C" subissaient environ 1 % de dégradation de l'acétate de benzyle qui est très fortement odorant,
- une quasi-absence d'oxydes de limonène, de myrcène et d'ocimènes, produits d'oxydation des terpènes, alors que la composition C' subissait environ 40% de dégradation du limonène, et
- une oxydation très limitée de l'éthanol, se traduisant par la présence de 60 ppm d'acétaldéhyde DEA et l'absence d'acétate d'éthyle, alors que pour la composition C', 900 ppm d'acétaldéhyde DEA et 110 ppm d'acétate d'éthyle ont été mesurés.

Ces résultats montrent la bonne stabilité chimique des compositions selon l'invention, qui permettent d'éviter la formation de produits de dégradation des parfums, dont certains sont des allergènes potentiels.

### Exemple 3 : Etude comparative de la solubilisation du parfum

On a comparé l'aspect visuel de deux compositions de parfum C1 et C'1 correspondant respectivement à la composition C de l'Exemple 2 dans laquelle le parfum était l'extrait de parfum "Eau de Lancôme" et à la composition C' de l'Exemple 2 renfermant le même extrait de parfum.

La composition C'1 présentait un aspect trouble dès sa préparation. Au contraire, la composition C1 se présentait sous la forme d'un mélange parfaitement clair et homogène, stable deux mois à température ambiante et à 45°C.

Cet essai illustre la capacité des mélanges éthanol-silicone selon l'invention à solubiliser de fortes concentrations de parfums, par rapport aux solutions hydroalcooliques classiques.

### Exemple 4 : Compositions de parfum

Les compositions ci-dessous peuvent être préparées de manière classique pour l'homme du métier.

| Composition A | |
|---|---|
| Extrait de parfum "Noa" | 15 % |
| Décaméthyltétrasiloxane | 10 % |
| Ethanol | 75 % |

| Composition B | |
|---|---|
| Extrait de parfum "Emporio White She" | 20 % |
| Décaméthyltétrasiloxane | 15 % |
| Ethanol | 65 % |

| Composition C | |
|---|---|
| Extrait de parfum "Attraction" | 30 % |
| Décaméthyltétrasiloxane | 5 % |
| Ethanol | 65 % |

## Revendications

1. Composition de parfum comprenant : (a) de 11 à 60% en poids d'un mélange de matières odoriférantes, (b) de 51 à 80% en poids d'éthanol, (c) de 3 à 30% en poids de décaméthyltétrasiloxane, par rapport au poids total de la composition.

2. Composition selon la revendication 1, **caractérisée en ce qu'**elle renferme de 11 à 25% en poids de matières odoriférantes.

3. Composition selon la revendication 1 ou 2, **caractérisée en ce qu'**elle renferme de 51 à 65% d'éthanol.

4. Composition selon l'une quelconque des revendications 1 à 3, **caractérisée en ce qu'**elle renferme de 10 à 30% en poids de décaméthyltétrasiloxane.

5. Composition selon l'une quelconque des revendications 1 à 4, **caractérisée en ce qu'**elle renferme en outre de l'eau, en quantité ne dépassant pas 7% du poids total de la composition.

6. Composition selon l'une quelconque des revendications 1 à 5, **caractérisée en ce qu'**elle ne contient pas d'alkyldiméthylsiloxane dont le groupe alkyle contient de 2 à 13 atomes de carbone.

7. Composition selon l'une quelconque des revendications précédentes, **caractérisée en ce qu'**elle renferme moins de 0,5% de tensioactif.

8. Composition selon l'une quelconque des revendications 1 à 7, **caractérisée en ce qu'**elle est conditionnée dans un dispositif aérosol.

## Claims

1. Fragrance composition comprising: (a) from 11 to 60% by weight of a mixture of odoriferous materials, (b) from 51 to 80% by weight of ethanol and (c) from 3 to 30% by weight of decamethyltetrasiloxane, with respect to the total weight of the composition.

2. Composition according to Claim 1, **characterized in that** it includes from 11 to 25% by weight of odoriferous materials.

3. Composition according to Claim 1 or 2, **characterized in that** it includes from 51 to 65% of ethanol.

4. Composition according to any one of Claims 1 to 3, **characterized in that** it includes from 10 to 30% by weight of decamethyltetrasiloxane.

5. Composition according to any one of Claims 1 to 4, **characterized in that** it additionally includes water in an amount not exceeding 7% of the total weight of the composition.

6. Composition according to any one of Claims 1 to 5, **characterized in that** it does not comprise alkyldimethylsiloxane in which the alkyl group comprises from 2 to 13 carbon atoms.

7. Composition according to any one of the preceding claims, **characterized in that** it includes less than 0.5% of surfactant.

8. Composition according to any one of Claims 1 to 7, **characterized in that** it is packaged in an aerosol device.

## Patentansprüche

1. Parfümzusammensetzung, die enthält:
(a) 11 bis 60 Gew.-% eines Gemisches von Duftstoffen,
(b) 51 bis 80 Gew.-% Ethanol und
(c) 3 bis 30 Gew.-% Decamethyltetrasiloxan,
bezogen auf das Gesamtgewicht der Zusammensetzung.

2. Zusammensetzung nach Anspruch 1, **dadurch gekennzeichnet, dass** sie 11 bis 25 Gew.-% Duftstoffe enthält.

3. Zusammensetzung nach Anspruch 1 oder 2, **dadurch gekennzeichnet, dass** sie 51 bis 65 % Ethanol enthält.

4. Zusammensetzung nach einem der Ansprüche 1 bis 3, **dadurch gekennzeichnet, dass** sie 10 bis 30 Gew.-% Decamethyltetrasiloxan enthält.

5. Zusammensetzung nach einem der Ansprüche 1 bis 4, **dadurch gekennzeichnet, dass** sie ferner Wasser in einer Menge von nicht mehr als 7 Gew.-% enthält, bezogen auf das Gesamtgewicht der Zusammensetzung.

6. Zusammensetzung nach einem der Ansprüche 1 bis 5, **dadurch gekennzeichnet, dass** sie kein Alkyldimethylsiloxan enthält, dessen Alkylgruppe 2 bis 13 Kohlenstoffatome aufweist.

7. Zusammensetzung nach einem der vorhergehenden Ansprüche, **dadurch gekennzeichnet, dass** sie weniger als 0,5 % Tensid enthält.

8. Zusammensetzung nach einem der Ansprüche 1 bis 7, **dadurch gekennzeichnet, dass** sie als Aerosolvorrichtung konfektioniert ist.
